# EUROPÄISCHE PATENTSCHRIFT

(11) **EP 2 866 895 B1**
(45) Veröffentlichungstag und Bekanntmachung des Hinweises auf die Patenterteilung: **31.08.2016**
(21) Anmeldenummer: 13726187.1
(22) Anmeldetag: 29.05.2013
(51) Int. Cl.: A61Q 5/02, A61Q 19/10, A61K 8/42, A61K 8/34, C11D 1/52

(54) **TENSIDLÖSUNGEN MIT N-METHYL-N-OLEYLGLUCAMIN UND N-METHYL-N-C12-C14-ACYLGLUCAMIN**
SURFACTANT SOLUTIONS CONTAINING N-METHYL-N-OLEYLGLUCAMINES AND N-METHYL-N-C12-C14-ACYLGLUCAMINES
SOLUTIONS TENSIOACTIVES CONTENANT DES N-MÉTHYL-N-OLEYLGLUCAMINES ET DES N-MÉTHYL-N-C12-C14-ACYLGLUCAMINES

(30) Priorität: 30.05.2012 DE 102012010701
(43) Veröffentlichungstag der Anmeldung: 06.05.2015
(73) Patentinhaber: CLARIANT INTERNATIONAL LTD, 4132 Muttenz (CH)
(72) Erfinder: KLUG, Peter, 63762 Grossostheim (DE); SCHERL, Franz-Xaver, 84508 Burgkirchen (DE); MILDNER, Carina, 65931 Frankfurt am Main (DE); KEITZL, Eva-Maria, 84453 Mühldorf (DE)
(74) Vertreter: Holmes, Rosalind
(86) Internationale Anmeldenummer: PCT/EP2013/061044
(87) Internationale Veröffentlichungsnummer: WO 2013/178668

(56) Entgegenhaltungen:
- EP-A1- 0 709 449
- US-A- 5 750 748
- FRIEDRICH VOGEL: "Kosmetik aus der Sicht des Chemikers", CHEMIE IN UNSERER ZEIT, Nr. 5, 1. Januar 1986 (1986-01-01), Seiten 156-164, XP055109030, DOI: 10.1002/ciuz.19860200504

## Beschreibung

Die Erfindung betrifft Tensidlösungen enthaltend N-Methyl-N-oleylglucamin und N-Methyl-N-C₁₂-C₁₄-acylglucamine sowie kosmetische Zusammensetzungen enthaltend diese N-Methyl-N-acylglucamine.

Es ist bekannt, kurzkettige Zuckertenside als Solubilisatoren oder Tenside in Reinigungsmitteln oder kosmetischen Zusammensetzungen einzusetzen.

WO 95/17880 offenbart eine Haarshampoo-Zusammensetzung enthaltend Alkylglykolethersulfate und Alkylsulfate sowie Polyhydroxyalkylfettsäureamide. Als Alkylglykolethersulfat wird u.a. Lauryltriethylenglykolethersulfat, als Alkylsulfat wird u.a. Laurylsulfat genannt. Als Polyhydroxyalkylfettsäureamide werden Verbindungen der allgemeinen Formel

R²-CO-NR¹-Z

aufgeführt, wobei R¹ vorzugsweise C₁-C₄-Alkyl, insbesondere Methyl, R² vorzugsweise geradkettiges C₇C₁₉-Alkyl oder -Alkenyl, insbesondere geradkettiges C₁₁-C₁₆-Alkyl oder -Alkenyl, und Z insbesondere 1-Deoxyglucityl, 2-Deoxyfructityl, 1-Deoxymaltityl, 1-Deoxylactityl, 1-Deoxygalactityl, 1-Deoxymannityl oder 1-Deoxymaltotriothityl ist. Die Beispiele offenbaren Haarshampoo-Zusammensetzungen enthaltend Ammoniumlaurylsulfat, Ammoniumlauryltriethylenglykolsulfat und Lauryl-N-methylglucamid.

WO 95/19415 offenbart Gemische aus a) N-Acyl-N-methylglucaminen mit einem C₈-C₁₀-Acylrest und b) N-Acyl-N-methylglucaminen mit einem C₁₂-C₁₈-Acylrest, insbesondere einem C₁₂-C₁₄-Acylrest. Die Komponenten a) und b) sind in den Gemischen im Verhältnis a) zu b) von 80 : 20 bis 20 : 80, insbesondere im Verhältnis von 25 : 75 bis 40 : 60 enthalten. Die Tensidgemische sollen verbesserte oberflächenaktive Eigenschaften aufweisen und können weitere anionische, nichtionische, kationische und amphotere Tenside enthalten. Offenbart wird die Verwendung in Haarshampoos, Haarlotionen und Schaumbädern.

Glucamine sind Feststoffe mit Schmelzpunkten von ca. 85 °C (C₁₂-C₁₄-Acylglucamine, enthaltend 10 Gew.-% Propylenglykol) und ca. 85 °C (C₁₆-C₁₈-Acylglucamine enthaltend 20 Gew.-% Propylenglykol). Die entsprechenden Reinsubstanzen weisen sogar noch bedeutend höhere Schmelzpunkte auf. C₁₂-C₁₄-Acylglucamine bilden bei Verdünnung mit Wasser im Allgemeinen schlecht wasserlösliche Gele. So führt die Verdünnung dieser N-Acyl-N-methylglucamine in Wasser bis herunter zu 2 gew.-%igen Lösungen zur Bildung einer Gelphase, was die Handhabung dieser Tenside stark erschwert.

N-Methyl-N-acylglucamine weisen die Formel (I) auf, 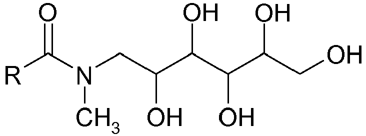 worin R einen Alkyl- oder ein- oder mehrfach ungesättigten Alkenylrest bedeutet.

Aufgabe der Erfindung ist es, konzentrierte Tensidlösungen enthaltend Acylglucamine bereitzustellen, die beim Verdünnen mit Wasser keine Gelbildung verursachen.

Gelöst wird die Aufgabe durch eine Tensidlösung enthaltend
(a) 28 bis 65 Gew.-% eines Gemischs aus
   (a1) 5 bis 20 Gew.-% N-Methyl-N-oleylacylglucaminen,
   (a2) 50 bis 95 Gew.-% N-Methyl-N-C₁₂-C₁₄-acylglucaminen,
   (a3) 0 bis 30 Gew.-% weiteren N-Methyl-N-acylglucaminen,
   wobei die Summe der Komponenten (a1), (a2) und (a3) 100 Gew.-% ergibt,
(b) 0 bis 20 Gew.-% eines oder mehrerer Alkohole,
(c) 20 bis 72 Gew.-% Wasser,
(d) 0 bis 5 Gew.-% Additive,
wobei die Summe der Komponenten (a), (b), (c) und (d) 100 Gew.-% ergibt.

Es wurde gefunden, dass ein Gemisch aus N-Methyl-N-oleylglucaminen und N-Methyl-N-C₁₂-C₁₄-acylglucaminen beim Verdünnen mit Wasser keine Gelbildung verursacht. Dadurch lassen sich aus den erfindungsgemäßen Tensidlösungen hergestellte verdünnte Lösungen im Formulierungsprozess leichter mit den weiteren Inhaltsstoffen kosmetischer Zusammensetzungen vermischen und homogenisieren. Die erhaltenen kosmetischen Zusammensetzungen weisen keine Inhomogenitäten auf. Außerdem werden die Rührzeiten bei der Herstellung der kosmetischen Zusammensetzungen verringert.

Die erfindungsgemäßen Tensidlösungen weisen im Allgemeinen einen Schmelzpunkt von < 40 °C auf und sind damit in angewärmtem Zustand pumpbar und im industriellen Maßstab leicht handhabbar.

Die in den erfindungsgemäßen Tensidlösungen enthaltenen N-Methyl-N-acylglucamine enthalten 5 bis 20 Gew.-% N-Methyl-N-acylglucamine, die eine Oleylgruppe enthalten. Bevorzugt liegt der Anteil an N-Methyl-N-acylglucaminen, die eine Oleylgruppe enthalten, bei 6 bis 18 Gew.-%, besonders bevorzugt bei 7 bis 15 Gew.-%, bezogen auf die Menge aller N-Methyl-N-acylglucamine.

Daneben enthalten die N-Methyl-N-acylglucamine 50 bis 95 Gew.-% N-Methyl-N-acylglucamine, die eine C₁₂-C₁₄-Acylgruppe enthalten. Diese sind von Laurin- und Myristinsäure abgeleitet. Bevorzugt liegt dieser Anteil bei 60 bis 90 Gew.-%, besonders bevorzugt bei 70 bis 85 Gew.-%, bezogen auf die Menge aller N-Methyl-N-acylglucamine.

Weiterhin enthalten die in den erfindungsgemäßen Tensidlösungen enthaltenen N-Methyl-N-acylglucamine 0 bis 30 Gew.-% weitere, von kurzkettigen und/oder langkettigen Fettsäuren abgeleitete N-Methyl-N-acylglucaminen, insbesondere solche, welche C₁-C₄-Acyl, C₆-Acyl, C₈-Acyl, C₁₀-Acyl, C₁₆-Acyl, C₁₈-Acyl (außer Oleyl) und/oder C₂₀-Acyl enthalten. Bevorzugt beträgt dieser Anteil 5 bis 25 Gew.-%, besonders bevorzugt 7 bis 20 Gew.-%, bezogen auf die Menge aller N-Methyl-N-acylglucamine.

Die N-Methyl-N-acylglucamine können, wie in EP 0 550 637 B1 beschrieben, durch Umsetzung der entsprechenden Fettsäureester bzw. Fettsäureestergemische mit N-Methylglucamin in Gegenwart eines Hydroxylgruppen oder Alkoxygruppen aufweisenden Lösungsmittels hergestellt werden. Geeignete Lösungsmittel sind beispielsweise C₁-C₄-Monoalkohole, Ethylenglykol, 1,2-Propylenglykol, Glycerin sowie alkoxylierte Alkohole. Bevorzugt ist 1,2-Propylenglykol. N-Methylglucamin kann, wie ebenfalls in EP 0 550 637 A1 beschrieben, durch reduktive Aminierung von Glukose mit Methylamin erhalten werden.

Geeignete Fettsäureester, die mit den N-Methylglucaminen zu N-Methyl-N-acylglucaminen umgesetzt werden, sind im Allgemeinen die Methylester, die durch Umesterung aus natürlichen Fetten und Ölen, beispielsweise den Triglyceriden, gewonnen werden.

Geeignete Rohstoffe für die Herstellung der Fettsäuremethylester sind beispielsweise Kokosöl oder Palmöl.

Daneben können die erfindungsgemäßen Tensidlösungen als Komponente (b) einen oder mehrere Alkohole enthalten. Geeignete Alkohole sind mit Wasser mischbare Monoalkohole oder Diole. Bevorzugt sind Ethanol, 1,2-Propylenglykol, Glycerin, 1,3-Propylenglykol und Isopropanol.

In einer bevorzugten Ausführungsform der Erfindung weisen die Tensidlösungen keinen Monoalkohol auf. Sie können aber Diole wie 1,2-Propylenglykol enthalten.

Als Additive können in den erfindungsgemäßen Tensidlösungen enthalten sein: Konservierungsmittel, Komplexierungsmittel sowie Neutralisationsmittel und Puffer wie z.B. Zitronensäure oder Zitronensäuresalze.

In einer bevorzugten Ausführungsform enthält die erfindungsgemäße Tensidlösung
(a) 30 bis 65 Gew.-% eines Gemischs aus
   (a1) 6 bis 18 Gew.-% N-Methyl-N-oleylglucamin,
   (a2) 60 bis 75 Gew.-% N-Methyl-N-C₁₂-C₁₄-acylglucamine,
   (a3) 10 bis 30 Gew.-% weiterer N-Methyl-N-acylglucamine,
   wobei die Summe der Komponenten (a1), (a2) und (a3) 100 Gew.-% ergibt,
(b) 3 bis 17 Gew.-% eines oder mehrerer Alkohole,
(c) 25 bis 67 Gew.-% Wasser,
(d) 0 bis 2 Gew.-% Additive,
wobei die Summe der Komponenten (a), (b), (c) und (d) 100 Gew.-% ergibt.

Gegenstand der Erfindung ist auch die Verwendung der Tensidlösungen zur Herstellung von kosmetischen Zusammensetzungen.

Die Herstellung der kosmetischen Zusammensetzungen umfasst dabei den Schritt des Verdünnens der Tensidlösungen mit Wasser. Im Allgemeinen werden die erfindungsgemäßen Tensidlösungen mit Wasser im Verhältnis 1 : 1 bis 1 : 50, bevorzugt 1 : 2 bis 1 : 10 verdünnt. Im Allgemeinen werden die Tensidlösungen dabei soweit verdünnt, dass die Endkonzentration der N-Methyl-N-acylglucamine im Bereich von 1 bis 10 Gew.-%, bevorzugt im Bereich von 2 bis 5 Gew.-% liegt.

Gegenstand der Erfindung sind auch konzentrierte Zusammensetzungen enthaltend
(A) 25 bis 60 Gew.-% eines Gemischs aus
   (a1) 5 bis 20 Gew.-%, bevorzugt 6 bis 18 Gew.-% N-Methyl-N-oleylglucamin,
   (a2) 50 bis 95 Gew.-%, bevorzugt 60 bis 75 Gew.-% N-Methyl-N-C₁₂-C₁₄-acylglucamine,
   (a3) 0 bis 30 Gew.-%, bevorzugt 10 bis 30 Gew.-% weiterer N-Methyl-N-acylglucamine,
   wobei die Summe der Komponenten (a1), (a2) und (a3) 100 Gew.-% ergibt, als Komponente (A),
(B) 0 bis 10 Gew.-% eines oder mehrerer weiterer Tenside als Komponente (B),
(C) 0 bis 20 Gew.-% eines oder mehrerer Alkohole als Komponente (C),
(D) 20 bis 72 Gew.-% Wasser als Komponente (D),
(E) 0 bis 5 Gew.-% weiterer Hilfs- und Zusatzstoffe als Komponente (E),
wobei die Summe der Komponenten (A), (B), (C), (D) und (E) 100 Gew.-% ergibt.

Vorzugsweise enthalten die Zusammensetzungen
(A) 25 bis 60 Gew.-% der Komponente (A),
(B) 1 bis 5 Gew.-% der Komponente (B),
(C) 3 bis 17 Gew.-% der Komponente (C),
(D) 25 bis 65 Gew.-% der Komponente (D),
(E) 0 bis 2 Gew.-% der Komponente (E),
wobei die Summe der Komponenten (A), (B), (C), (D) und (E) 100 Gew.-% ergibt.

Die weiteren Tenside (B) können nichtionischen Tenside, anionischen Tenside, kationischen Tenside und Betain-Tenside sein.

Als anionische Tenside in Betracht kommen (C₁₀-C₂₂)-Alkyl-und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, alpha-Sulfofettsäureester, Alkylbenzolsulfonate, Alkylphenolglykolethersulfonate, Sulfosuccinate, Sulfobernsteinsäurehalbester und -diester, Fettalkoholphosphate, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulfosuccinate, Sulforicinoleate, Acylglutamate und Acylglycinate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie der analogen Alkylammonium-Salze.

In einer Ausführungsform der Erfindung enthalten die Zusammensetzungen ein oder mehrere anionische Tenside aus der Gruppe der Alkylsulfate und Alkylethersulfate.

Bevorzugte Alkylsulfate sind die C₈-C₂₀-Alkylsulfate, insbesondere die linearen C₈-C₂₀-Alkylsulfate in Form ihrer Natrium-, Kalium- oder Ammoniumsalze. Beispiele für Alkylsulfate sind Laurylsulfat, Cocosalkylsulfat und Talgalkylsulfat. Besonders bevorzugt ist Laurylsulfat.

Bevorzugte Alkylethersulfate sind die C₈-C₂₀-Alkylethersulfate, besonders bevorzugt sind die linearen C₈-C₂₀-Alkylethersulfate, insbesondere die von den ethoxylierten Fettalkoholen abgeleiteten Alkylglykolethersulfate, in Form ihrer Natrium-, Kalium- oder Ammoniumsalze. Beispiele für Alkylethersulfate sind Laurylethersulfat, Cocosalkylethersulfat und Talgalkylethersulfat. Beispiele für Glykolethersulfate sind Lauryltriethylenglykolethersulfat, Cocosalkyltriethylenglykolethersulfat und Talgalkylhexaethylenglykolethersulfat. Insbesondere bevorzugt ist Laurylglykolethersulfat, beispielsweise Lauryltriethylenglykolethersulfat.

Betain-Tenside enthalten im selben Molekül eine kationische Gruppe, insbesondere eine Ammonium-Gruppe, und eine anionische Gruppe, die eine Carboxylat-Gruppe, SulfatGruppe oder Sulfonat-Gruppe sein kann. Geeignete Betaine sind Alkylbetaine wie Cocobetain oder Fettsäurealkylamidopropylbetaine, beispielsweise Cocosacylamidopropyldimethylbetain, C₁₂-C₁₈-Dimethylaminohexanoate oder C₁₀-C₁₈-Acylamidopropandimethylbetaine.

In einer bevorzugten Ausführungsform der Erfindung enthalten die Zusammensetzungen ein oder mehrere Amidopropylbetaine der allgemeinen Formel (I) 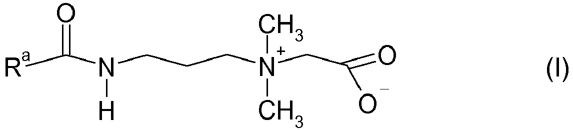 worin R^{a} eine lineare oder verzweigte gesättigte C₇-C₂₁-Alkylgruppe oder eine lineare oder verzweigte ein- oder mehrfach ungesättigte C₇-C₂₁-Alkenylgruppe ist.

In einer weiteren bevorzugten Ausführungsform der Erfindung enthalten die Zusammensetzungen ein oder mehrere Betaine der Formel (II) 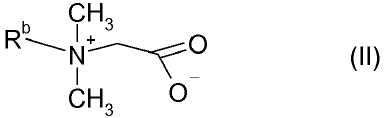 worin R^{b} eine lineare oder verzweigte gesättigte C₈-C₂₂-Alkylgruppe oder eine lineare oder verzweigte ein- oder mehrfach ungesättigte C₈-C₂₂-Alkenylgruppe ist.

In einer weiteren bevorzugten Ausführungsform der Erfindung enthalten die Zusammensetzungen ein oder mehrere Sulfobetaine der Formel (III) 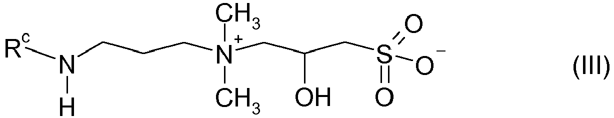 worin R^{c} eine lineare oder verzweigte gesättigte C₈-C₂₂-Alkylgruppe oder eine lineare oder verzweigte ein- oder mehrfach ungesättigte C₈-C₂₂-Alkenylgruppe ist.

Besonders bevorzugt enthalten die Zusammensetzungen ein oder mehreren Betaintenside ausgewählt aus der Gruppe der Verbindungen bestehend aus den Amidopropylbetainen der Formel (I), den Betainen der Formel (II) und den Sulfobetainen der Formel (III).

In einer insbesondere bevorzugten Ausführungsform der Erfindung enthalten die Zusammensetzungen ein oder mehrere Betaintenside ausgewählt aus den Amidopropylbetainen der Formel (I).

In einer weiteren insbesondere bevorzugten Ausführungsform der Erfindung enthalten die Zusammensetzungen ein oder mehrere Betaintenside ausgewählt aus den Betainen der Formel (II).

In einer weiteren insbesondere bevorzugten Ausführungsform der Erfindung enthalten die Zusammensetzungen ein oder mehrere Betaintenside ausgewählt aus den Sulfobetainen der Formel (III).

Vorzugsweise ist der Rest R^{a} in dem einen oder den mehreren Amidopropylbetainen der Formel (I) eine lineare oder verzweigte gesättigte C₇-C₁₇-Alkylgruppe. Unter den linearen und verzweigten gesättigten Alkylgruppen R^{a} sind die linearen gesättigten Alkylgruppen bevorzugt.

Besonders bevorzugt handelt es sich bei den Amidopropylbetainen der Formel (I) um Cocamidopropylbetaine.

Vorzugsweise ist der Rest R^{b} in dem einen oder den mehreren Betainen der Formel (II) eine lineare oder verzweigte gesättigte C₈-C₁₈-Alkylgruppe und besonders bevorzugt eine lineare oder verzweigte gesättigte C₁₂-C₁₈-Alkylgruppe. Unter den linearen und verzweigten gesättigten Alkylgruppen R^{b} sind die linearen gesättigten Alkylgruppen bevorzugt.

Vorzugsweise ist der Rest R^{c} in der einen oder den mehreren Sulfobetainen der Formel (III) eine lineare oder verzweigte gesättigte C₈-C₁₈-Alkylgruppe und besonders bevorzugt eine lineare oder verzweigte gesättigte C₁₂-C₁₈-Alkylgruppe. Unter den linearen und verzweigten gesättigten Alkylgruppen R^{c} sind die linearen gesättigten Alkylgruppen bevorzugt.

Besonders bevorzugt enthalten die wässrigen Tensidlösungen Amidopropylbetaine der Formel (I) und/oder Alkylbetaine der Formel (II).

Vorzugsweise enthalten die Zusammensetzungen neben dem anionischen Tensid ein Betain-Tensid.

Besonders bevorzugt enthalten die Zusammensetzungen die oben beschriebenen Alkylsulfate und/oder Alkylethersulfate und Betaintenside.

Geeignete kationische Tenside sind substituierte oder unsubstituierte geradkettige oder verzweigte quartäre Ammoniumsalze vom Typ R¹N(CH₃)₃X, R¹R²N(CH₃)₂X, R¹R²R³N(CH₃)X oder R¹R²R³R⁴NX. Die Reste R¹, R², R³ und R⁴ können vorzugsweise unabhängig voneinander unsubstituiertes Alkyl mit einer Kettenlänge zwischen 8 und 24 C-Atomen, insbesondere zwischen 10 und 18 C-Atomen, Hydroxyalkyl mit 1 bis 4 C-Atomen, Phenyl, C₂- bis C₁₈-Alkenyl, C₇- bis C₂₄-Aralkyl, (C₂H₄O)ₓH, wobei x von 1 bis 3 bedeutet, ein oder mehrere Estergruppen enthaltende Alkylreste oder cyclische quartäre Ammoniumsalze sein. X ist ein geeignetes Anion. Bevorzugt sind (C₈-C₂₂)-Alkyltrimethylammoniumchlorid oder -bromid, besonders bevorzugt Cetyltrimethylammoniumchlorid oder -bromid, Di-(C₈-C₂₂)-Alkyldimethylammoniumchlorid oder -bromid, (C₈-C₂₂)-Alkyldimethylbenzylammoniumchlorid oder -bromid, (C₈-C₂₂)-Alkyl-dimethylhydroxyethylammoniumchlorid, -phosphat, -sulfat, -lactat, besonders bevorzugt Distearyldimethylammoniumchlorid, Di(C₈-C₂₂)-Alkylamidopropyltrimethylammoniumchlorid und -methosulfat.

Als nichtionische Tenside kommen beispielsweise folgende Verbindungen in Frage:
- Polyethylen-, Polypropylen- und Polybutylenoxidkondensate von Alkylphenolen. Diese Verbindungen umfassen die Kondensationsprodukte von Alkylphenolen mit einer C₆- bis C₂₀-Alkylgruppe, die entweder linear oder verzweigt sein kann, mit Alkenoxiden. Diese Tenside werden als Alkylphenolalkoxylate, z.B. Alkylphenolethoxylate, bezeichnet.
- Kondensationsprodukte von aliphatischen Alkoholen mit 1 bis 25 mol Ethylenoxid. Die Alkyl- oder Alkenylkette der aliphatischen Alkohole kann linear oder verzweigt, primär oder sekundär sein, und enthält im Allgemeinen 8 bis 22 Kohlenstoffatome. Besonders bevorzugt sind die Kondensationsprodukte von C₁₀-bis C₂₀-Alkoholen mit 2 bis 18 mol Ethylenoxid pro mol Alkohol. Die Alkoholethoxylate können eine enge ("Narrow Range Ethoxylates") oder eine breite Homologenverteilung des Ethylenoxides ("Broad Range Ethoxylates") aufweisen. Beispiele von kommerziell erhältlichen nichtionischen Tensiden dieses Typs sind Tergitol^{®} 15-S-9 (Kondensationsprodukt eines linearen sekundären C₁₁-C₁₅-Alkohols mit 9 mol Ethylenoxid), Tergitol^{®} 24-L-NMW (Kondensationsprodukt eines linearen primären C₁₂-C₁₄-Alkohols mit 6 mol Ethylenoxid bei enger Molgewichtsverteilung). Ebenfalls unter diese Produktklasse fallen die Genapol^{®}-Marken der Clariant.
- Kondensationsprodukte von Ethylenoxid mit einer hydrophoben Basis, gebildet durch Kondensation von Propylenoxid mit Propylenglykol. Der hydrophobe Teil dieser Verbindungen weist bevorzugt ein Molekulargewicht zwischen 1500 und 1800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von ca. 50 % des Gesamtgewichtes des Kondensationsproduktes, was einer Kondensation mit bis zu ca. 40 mol Ethylenoxid entspricht. Kommerziell erhältliche Beispiele dieser Produktklasse sind die Pluronic^{®}-Marken der BASF und die Genapol^{®} PF-Marken der Clariant.
- Kondensationsprodukte von Ethylenoxid mit einem Reaktionsprodukt von Propylenoxid und Ethylendiamin. Die hydrophobe Einheit dieser Verbindungen besteht aus dem Reaktionsprodukt von Ethylendiamin mit überschüssigem Propylenoxid und weist im Allgemeinen ein Molekulargewicht von 2500 bis 3000 auf. An diese hydrophobe Einheit wird Ethylenoxid bis zu einem Gehalt von 40 bis 80 Gew.-% Polyoxyethylen und einem Molekulargewicht von 5000 bis 11000 addiert. Kommerziell erhältliche Beispiele dieser Verbindungsklasse sind die Tetronic^{®}-Marken der BASF und die Genapol^{®} PN-Marken der Clariant.

Weitere geeignete nichtionische Tenside sind Alkyl- und Alkenyloligoglycoside sowie Fettsäurepolyglykolester oder Fettaminpolyglykolester mit jeweils 8 bis 20, vorzugsweise 12 bis 18 C-Atomen im Fettalkylrest, Alkyloligoglycoside, Alkenyloligoglycoside und Fettsäure-N-alkylglucamine.

Daneben können die erfindungsgemäßen Tensidlösungen als Komponente (C) einen oder mehrere Alkohole enthalten, die aus der erfindungsgemäßen Tensidlösung stammen. Geeignete Alkohole sind die oben genannten, mit Wasser mischbaren Alkohole. In einer bevorzugten Ausführungsform der Erfindung enthalten die Zusammensetzungen keine Monoalkohole.

Hilfs- und Zusatzstoffe (E) sind beispielsweise Konservierungsmittel, Duftstoffe, Farbstoffe und Rückfettungsmittel.

Als Konservierungsmittel eignen sich die in dem betreffenden Anhang der europäischen Kosmetikgesetzgebung gelisteten Konservierungsmittel, beispielsweise Phenoxyethanol, Benzylalkohol, Parabene, Benzoesäure und Sorbinsäure, besonders gut geeignet ist beispielsweise 1,3-Bis(hydroxymethyl)-5,5-dimethylimidazolidin-2,4-dion (Nipaguard^{®} DMDMH).

Die Menge der Konservierungsmittel in den erfindungsgemäßen Zusammensetzungen beträgt im Allgemeinen von 0 bis 2 Gew.-%, bezogen auf das Gesamtgewicht der fertigen Zusammensetzungen.

In einer bevorzugten Ausführungsform der Erfindung liegen die erfindungsgemäßen Zusammensetzungen in Form von Konzentraten zur Herstellung von Produkten zur Haar- und Hautreinigung wie Haarshampoos, Duschbäder, Handseifen und Gesichtsreiniger vor.

Die Erfindung wird durch die nachstehenden Beispiele näher erläutert.

### Beispiele

### Beispiele 1 bis 4 und Vergleichsbeispiel 1

Die im folgenden beschriebenen N-Acyl-N-methylglucamine wurden nach EP 0 550 637 aus den korrespondierenden Fettsäuremethylestern oder Triglyceriden und N-Methylglucamin in Gegenwart von 1,2-Propylenglykol als Lösemittel hergestellt und als Feststoff bestehend aus Aktivsubstanz und 1,2-Propylenglykol erhalten (alle Angaben in Gew.-%).

**Tabelle 1**

| Herstellbeispiel | Methylester | Triglycerid | Aktivsubstanz (%) | 1,2 Propylenglykol (%) | Schmelzpunkt (°C) |
|---|---|---|---|---|---|
| 1 | C12/14 (C12: 70 %, C14 30 %) | - | 90 | 10 | 85 |
| 2 | | Cocosöl (C8: 6 %; C10:6%; C12: 48 % C14: 20 % C16: 10 %; | 82 | 10 (+ 8 % Glycerin) | 50 |
| | | C18: 2 %, C18'=8%) | | | |
| 3 | Cocosfettsäuremethylester (C8: 6 %; C 10: 6 %; C12: 48 % C14: 20 % C16: 10 %; C18: 2 %, C18'= 8 %) | | 90 | 10 | 75 |

| | | | | | |
|---|---|---|---|---|---|
| C18 = Stearinsäuremethylester; C18' = Ölsäuremethylester | | | | | |

Die obigen Produkt sind schwer zu handhaben und weisen Schmelzpunkte größer 50 °C auf. Sie wurden deshalb mit Wasser und Ethanol verdünnt, um unter 50 °C als Flüssigkeit handhabbar zu sein (alle Angaben in Gew.-%).

**Tabelle 2**

| Beispiel | Glucamid nach | Aktivgehalt Glucamine | Wasser | Ethanol | Propylenglykol | Schmelzpunkt der Tensidmischung (°C) | 10 % in Wasser | 5 % in Wasser | 2 % in Wasser |
|---|---|---|---|---|---|---|---|---|---|
| Vergleichsbeispiel 1 | Herstellbeispiel 1 | 60 | 23 | 10 | 7 | 40 | Weißes Gel | Weißes Gel | Gel/Flüssigkeit |
| Beispiel 1 | Herstellbeispiel 2 | 60 | 20 | 10 | 7 (+ 3 % Glycerin) | 32,5 | Klare Flüssigkeit ca. 100 mPas | Klare Flüssigkeit | Klare Flüssigkeit |
| Beispiel 2 | Herstellbeispiel 2 | 30 | 65 | 0 | 3 (+ 2 % Glycerin) | 35 | Klare Flüssigkeit ca. 100 mPas | Klare Flüssigkeit | Klare Flüssigkeit |
| Beispiel 3 | Herstellbeispiel 3 | 55 | 28 | 10 | 7 | 32,5 | Klare Flüssigkeit ca. 1000 mPas | Klare Flüssigkeit, ca 100 mPas | Klare Flüssigkeit |
| Beispiel 4 | Herstellbeispiel 3 | 30 | 67 | 0 | 3 | 38 | Klare Flüssigkeit ca. 1000 mPas | Klare Flüssigkeit, ca 100 mPas | Klare Flüssigkeit |

Wie aus Tabelle 2 ersichtlich, sind die erfindungsgemäßen Zusammensetzungen der Beispiele 1 bis 4 im Gegensatz zu der Zusammensetzung gemäß Vergleichsbeispiel 1 nicht nur gut handhabbar, sondern auch in Wasser leicht und ohne Gelbildung verdünnbar.

## Patentansprüche

1. Tensidlösung enthaltend
(a) 28 bis 65 Gew.-% eines Gemischs aus
(a1) 5 bis 20 Gew.-% N-Methyl-N-oleylglucamin,
(a2) 50 bis 93 Gew.-% N-Methyl-N-C₁₂-C₁₄-acylglucamine,
(a3) 0 bis 30 Gew.-% weiterer N-Methyl-N-acylglucamine,
wobei die Summe der Komponenten (a1), (a2) und (a3) 100 Gew.-% ergibt,
(b) 0 bis 20 Gew.-% eines oder mehrerer Alkohole,
(c) 20 bis 72 Gew.-% Wasser,
(d) 0 bis 5 Gew.-% Additive,
wobei die Summe der Komponenten (a), (b), (c) und (d) 100 Gew.-% ergibt.

2. Tensidlösung nach Anspruch 1, enthaltend als Komponente (b) einen oder mehrere Alkohole ausgewählt aus der Gruppe bestehend aus Ethanol, Isopropanol, 1,2-Propylenglykol, 1,3-Propylenglykol und Glycerin.

3. Tensidlösung nach Anspruch 1 oder 2, enthaltend
(a) 30 bis 65 Gew.-% eines Gemischs aus
(a1) 6 bis 18 Gew.-% N-Methyl-N-oleylglucamin,
(a2) 60 bis 75 Gew.-% N-Methyl-N-C₁₂-C₁₄-acylglucamine,
(a3) 10 bis 30 Gew.-% weiterer N-Methyl-N-acylglucamine,
wobei die Summe der Komponenten (a1), (a2) und (a3) 100 Gew.-% ergibt,
(b) 3 bis 17 Gew.-% eines oder mehrerer Alkohole,
(c) 25 bis 67 Gew.-% Wasser,
(d) 0 bis 2 Gew.-% Additive,
wobei die Summe der Komponenten (a), (b), (c) und (d) 100 Gew.-% ergibt.

4. Tensidlösung nach einem der Ansprüche 1 bis 3, **dadurch gekennzeichnet, dass** sie keinen Monoalkohol enthält.

5. Verwendung einer Tensidlösung nach einem der Ansprüche 1 bis 4 zur Herstellung von kosmetischen Zusammensetzungen.

6. Verfahren zur Herstellung von kosmetischen Zusammensetzungen, umfassend den Schritt des Verdünnens der Tensidlösung nach einem der Ansprüche 1 bis 4 mit Wasser.

7. Zusammensetzungen enthaltend
(A) 25 bis 60 Gew.-% eines Gemischs aus
(a1) 5 bis 20 Gew.-% N-Methyl-N-oleylglucamin,
(a2) 50 bis 95 Gew.-% N-Methyl-N-C₁₂-C₁₄-acylglucamine,
(a3) 0 bis 30 Gew.-% weiterer N-Methyl-N-acylglucamine,
wobei die Summe der Komponenten (a1), (a2) und (a3) 100 Gew.-% ergibt, als Komponente (A),
(B) 0 bis 10 Gew.-% eines oder mehrerer weiterer Tenside als Komponente (B),
(C) 0 bis 20 Gew.-% eines oder mehrerer Alkohole als Komponente (C),
(D) 20 bis 72 Gew.-% Wasser als Komponente (D),
(E) 0 bis 5 Gew.-% weiterer Hilfs- und Zusatzstoffe als Komponente (E),
wobei die Summe der Komponenten (A), (B), (C), (D) und (E) 100 Gew.-% ergibt.

8. Zusammensetzungen nach Anspruch 7 enthaltend
(a) 25 bis 60 Gew.-% der Komponente (A),
(b) 1 bis 5 Gew.-% der Komponente (B),
(c) 3 bis 17 Gew.-% der Komponente (C),
(d) 25 bis 65 Gew.-% der Komponente (D),
(e) 0 bis 2 Gew.-% der Komponente (E),
wobei die Summe der Komponenten (A), (B), (C), (D) und (E) 100 Gew.-% ergibt.

## Claims

1. A surfactant solution comprising
(a) 28% to 65% by weight of a mixture of
(a1) 5% to 20% by weight of N-methyl-N-oleylglucamine,
(a2) 50% to 93% by weight of N-methyl-N-C₁₂-C₁₄-acylglucamines,
(a3) 0% to 30% by weight of further N-methyl-N-acylglucamines,
where the sum total of components (a1), (a2) and (a3) is 100% by weight,
(b) 0% to 20% by weight of one or more alcohols,
(c) 20% to 72% by weight of water,
(d) 0% to 5% by weight of additives,
where the sum total of components (a), (b), (c) and (d) is 100% by weight.

2. The surfactant solution as claimed in claim 1, comprising, as component (b), one or more alcohols selected from the group consisting of ethanol, isopropanol, 1,2-propylene glycol, 1,3-propylene glycol and glycerol.

3. The surfactant solution as claimed in claim 1 or 2, comprising
(a) 30% to 65% by weight of a mixture of
(a1) 6% to 18% by weight of N-methyl-N-oleylglucamine,
(a2) 60% to 75% by weight of N-methyl-N-C₁₂-C₁₄-acylglucamines,
(a3) 10% to 30% by weight of further N-methyl-N-acylglucamines,
where the sum total of components (a1), (a2) and (a3) is 100% by weight,
(b) 3% to 17% by weight of one or more alcohols,
(c) 25% to 67% by weight of water,
(d) 0% to 2% by weight of additives,
where the sum total of components (a), (b), (c) and (d) is 100% by weight.

4. The surfactant solution as claimed in any of claims 1 to 3, which does not comprise any monoalcohol.

5. The use of a surfactant solution as claimed in any of claims 1 to 4 for production of cosmetic compositions.

6. A process for producing cosmetic compositions, comprising the step of diluting the surfactant solution as claimed in any of claims 1 to 4 with water.

7. A composition comprising
(A) 25% to 60% by weight of a mixture of
(a1) 5% to 20% by weight of N-methyl-N-oleylglucamine,
(a2) 50% to 95% by weight of N-methyl-N-C₁₂-C₁₄-acylglucamines,
(a3) 0% to 30% by weight of further N-methyl-N-acylglucamines,
where the sum total of components (a1), (a2) and (a3) is 100% by weight, as component (A),
(B) 0% to 10% by weight of one or more further surfactants as component (B),
(C) 0% to 20% by weight of one or more alcohols as component (C),
(D) 20% to 72% by weight of water as component (D),
(E) 0% to 5% by weight of further auxiliaries and additives as component (E),
where the sum total of components (A), (B), (C), (D) and (E) is 100% by weight.

8. The composition as claimed in claim 7, comprising
(a) 25% to 60% by weight of component (A),
(b) 1 % to 5% by weight of component (B),
(c) 3% to 17% by weight of component (C),
(d) 25% to 65% by weight of component (D),
(e) 0% to 2% by weight of component (E),
where the sum total of components (A), (B), (C), (D) and (E) is 100% by weight.

## Revendications

1. Solution de tensioactifs, contenant
(a) 28 à 65 % en poids d'un mélange de
(a1) 5 à 20 % en poids de N-méthyl-N-oléylglucamine,
(a2) 50 à 93 % en poids de N-méthyl-N-acyl(C₁₂-C₁₄)glucamines,
(a3) 0 à 30 % en poids d'autres N-méthyl-N-acylglucamines,
la somme des composants (a1), (a2) et (a3) étant égale à 100 % en poids,
(b) 0 à 20 % en poids d'un ou de plusieurs alcools,
(c) 20 à 72 % en poids d'eau,
(d) 0 à 5 % en poids d'additifs,
la somme des composants (a), (b), (c) et (d) étant égale à 100 % en poids.

2. Solution de tensioactifs selon la revendication 1, contenant comme composant (b) un ou plusieurs alcools choisis dans le groupe constitué par l'éthanol, l'isopropanol, le 1,2-propylèneglycol, le 1,3-propylèneglycol et le glycérol.

3. Solution de tensioactifs selon la revendication 1 ou 2, contenant
(a) 30 à 65 % en poids d'un mélange de
(a1) 6 à 18 % en poids de N-méthyl-N-oléylglucamine,
(a2) 60 à 75 % en poids de N-méthyl-N-acyl(C₁₂-C₁₄)-glucamines,
(a3) 10 à 30 % en poids d'autres N-méthyl-N-acylglucamines,
la somme des composants (a1), (a2) et (a3) étant égale à 100 % en poids,
(b) 3 à 17 % en poids d'un ou de plusieurs alcools,
(c) 25 à 67 % en poids d'eau,
(d) 0 à 2 % en poids d'additifs,
la somme des composants (a), (b), (c) et (d) étant égale à 100 % en poids.

4. Solution de tensioactifs selon l'une quelconque des revendications 1 à 3, **caractérisée en ce qu'**elle ne contient aucun monoalcool.

5. Utilisation d'une solution de tensioactifs selon l'une quelconque des revendications 1 à 4, pour la production de compositions cosmétiques.

6. Procédé pour la production de compositions cosmétiques, comprenant l'étape de la dilution de la solution de tensioactifs selon l'une quelconque des revendications 1 à 4 avec de l'eau.

7. Compositions contenant
(A) 25 à 60 % en poids d'un mélange de
(a1) 5 à 20 % en poids de N-méthyl-N-oléylglucamine,
(a2) 50 à 95 % en poids de N-méthyl-N-acyl(C₁₂-C₁₄)glucamines,
(a3) 0 à 30 % en poids d'autres N-méthyl-N-acylglucamines,
la somme des composants (a1), (a2) et (a3) étant égale à 100 % en poids, en tant que composant (A),
(B) 0 à 10 % en poids d'un ou de plusieurs autres tensioactifs en tant que composant (B),
(C) 0 à 20 % en poids d'un ou de plusieurs alcools en tant que composant (C),
(D) 20 à 72 % en poids d'eau en tant que composant (D),
(E) 0 à 5 % en poids d'autres adjuvants et additifs en tant que composant (E),
la somme des composants (A), (B), (C), (D) et (E) étant égale à 100 % en poids.

8. Compositions selon la revendication 7, contenant
(a) 25 à 60 % en poids du composant (A),
(b) 1 à 5 % en poids du composant (B),
(c) 3 à 17 % en poids du composant (C),
(d) 25 à 65 % en poids du composant (D),
(e) 0 à 2 % en poids du composant (E),
la somme des composants (A), (B), (C), (D) et (E) étant égale à 100 % en poids.
